# EUROPÄISCHE PATENTSCHRIFT

(11) **EP 1 872 827 B1**
(45) Veröffentlichungstag und Bekanntmachung des Hinweises auf die Patenterteilung: **25.02.2009**
(21) Anmeldenummer: 07110677.7
(22) Anmeldetag: 20.06.2007
(51) Int. Cl.: A61N 5/10

(54) **Vorrichtung zur Bestrahlung von Gewebe mit mindestens einer Elektronenquelle sowie mit einer Mehrzahl von Strahlerköpfen**
Device for irradiating tissue with at least one electron source and with many radiation heads
Dispositif destiné à l'irradiation de tissus avec au moins une source d'électrons tout comme un grand nombre de têtes d'émetteurs de rayonnement

(30) Priorität: 26.06.2006 DE 102006029557; 23.04.2007 DE 102007019355
(43) Veröffentlichungstag der Anmeldung: 02.01.2008
(73) Patentinhaber: Forster, Jan, 85051 Ingolstadt (DE); Forster, Renate, 85051 Ingolstadt (DE); Müller, Reinhold G., 91080 Marloffstein (DE); Achterberg, Nils, 91233 Neunkirchen am Sand (DE)
(72) Erfinder: Forster, Jan, 85051, Ingolstadt (DE); Müller, Reinhold, 91080, Marloffstein (DE); Achterberg, Nils, 91233, Neunkirchen am Sand (DE)
(74) Vertreter: Bergmeier, Werner

(56) Entgegenhaltungen:
- EP-B1- 1 311 322
- WO-A-99/03397
- WO-A-20/04006269
- DE-A1- 19 736 192
- FR-A- 2 839 894

## Beschreibung

Die vorliegende Erfindung betrifft eine Vorrichtung zur Bestrahlung von Gewebe mit mindestens einer Elektronenquelle zur Erzeugung eines Elektronenstrahles sowie mit einer Mehrzahl von Strahlerköpfen, welche feststehend in einer Tragkonstruktion angeordnet sind. Die Strahlerköpfe sind hierbei ringförmig um ein Isozentrum der Vorrichtung angeordnet, in welchem das zu bestrahlende Gewebe platzierbar ist. Die Strahlerköpfe geben Strahlung in Richtung auf das Isozentrum ab. Weiterhin weist die Vorrichtung eine Strahlführung auf, mittels welcher der Elektronenstrahl in der Tragkonstruktion geführt und auf die Strahlerköpfe geleitet wird.

Geräte zur Bestrahlung von Gewebe sind im Stand der Technik hinreichend bekannt. Hierzu wird das zu bestrahlende Gewebe eines Patienten auf einem Patiententisch in einem Isozentrum der Bestrahlungsvorrichtung platziert. Die Bestrahlungsvorrichtung verfügt über eine Strahlenquelle, wobei die erzeugte Strahlung schließlich auf einen oder auch mehrere Strahlerköpfe in der Vorrichtung geführt wird. Üblicherweise werden die Strahlerköpfe schließlich mittels einer entsprechenden Vorrichtung um das Bestrahlungsobjekt rotiert, so dass die Strahlen zeitlich nacheinander immer aus verschiedenen Richtungen das Zielvolumen gelangen. Auf diese Weise erhält das Zielvolumen eine relativ große Dosis der Strahlung, während das umliegende gesunde Gewebe geschont wird. Nachteilig bei diesen bekannten Vorrichtungen ist es, dass die präzise Rotation der Strahlerköpfe wegen der Größe der zu bewegenden Massen technisch schwierig auszuführen ist.

Um den Aufbau einer Vorrichtung zur Bestrahlung von Gewebe zu vereinfachen, schlägt die EP 1 311 322 B1 vor, in einem Gehäuse eine Vielzahl von Strahlerköpfen feststehend anzuordnen. Vorgesehen ist eine gemeinsame Strahlenquelle für alle Strahlerköpfe, wobei ein Strahlungsteiler die Strahlung auf die verschiedenen Strahlerköpfe aufteilt. Nach der Aufteilung werden die einzelnen Strahlen in jeweils einer Strahlführung mehrfach umgelenkt bis sie schließlich auf die Strahlerköpfe geführt werden. Die Strahlerköpfe sind weitgehend ringförmig um das zu bestrahlende Gewebe angeordnet. Die Anordnung der Strahlerköpfe und der jeweils zugehörigen Strahlführungen führt bei dieser Vorrichtung zu einer vergleichsweise großen Ausdehnung des Gehäuses.

Aufgabe der vorliegenden Erfindung ist es, eine Vorrichtung zur Bestrahlung von Gewebe mit feststehenden Strahlerköpfen vorzuschlagen, welche einen einfachen und kompakten Aufbau aufweist.

Die Aufgabe wird gelöst mit den Merkmalen des Anspruches 1.

Eine Vorrichtung zur Bestrahlung von Gewebe weist eine Elektronenquelle zur Erzeugung eines Elektronenstrahles, eine Mehrzahl von Strahlerköpfen, welche feststehend in einer Tragkonstruktion und ringförmig um ein Isozentrum der Vorrichtung angeordnet sind, und eine Strahlführung auf. Mittels der Strahlführung wird der Elektronenstrahl in der Tragkonstruktion geführt und auf die Strahlerköpfe geleitet, welche Strahlung in Richtung auf das Isozentrum der Vorrichtung abgeben. Erfindungsgemäß ist die Strahlführung als Polygon ringförmig um das Isozentrum ausgebildet. An jedem Eckpunkt des Polygons sind hierbei Mittel zur Umlenkung des Elektronenstrahles angeordnet. Vorzugsweise ist für alle Strahlerköpfe ist eine gemeinsame Elektronenquelle vorgesehen, so dass nur ein Elektronenstrahl erzeugt wird, welcher nacheinander auf die einzelnen Strahlerköpfe geleitet wird.

Nach einer bevorzugten Ausführung der Erfindung ist die Strahlführung als symmetrisches Polygon ausgebildet. Vorzugsweise sind in der Vorrichtung fünf feststehende Strahlerköpfe vorgesehen, so dass die Strahlführung als Fünfeck ausgebildet ist. Ist für alle Strahlerköpfe eine gemeinsame Elektronenquelle vorgesehen, kann der Elektronenstrahl beispielsweise an einem Eckpunkt in die Strahlführung eingespeist werden und nacheinander auf die einzelnen Strahlerköpfe geleitet werden. Da die Strahlführung lediglich in einer Ebene ausgebildet ist, ist ein besonders kompakter Aufbau der Vorrichtung möglich. Die Tragkonstruktion weist hierdurch nur eine sehr geringe Ausdehnung in axialer Richtung auf. Da mit mehreren Strahlerköpfen zeitgleich oder auch sequentiell bestrahlt werden kann, ist eine Rotation der Strahler um das Zielvolumen nicht erforderlich. Bevorzugt ist ein sequentielles Betreiben der Strahlerköpfe vorgesehen, da dann keine Strahlungsteiler erforderlich sind. Hierbei können bereits während der Bestrahlung durch einen Strahlerkopf die Kollimatoren an den anderen Strahlerköpfen eingestellt werden, so dass kurze Bestrahlungszeiten erreicht werden können. Eine Unterbrechung des Elektronenstrahls ist nicht erforderlich. Durch den einfachen und schlanken Aufbau der Tragkonstruktion ist es weiterhin möglich, die Herstellkosten der Vorrichtung zu reduzieren.

Nach einer Weiterbildung der Erfindung ist es vorteilhaft, wenn die Mittel zur Umlenkung des Elektronenstrahles als Umlenkmagnete ausgebildet sind. Hierdurch ist eine einfache Führung des Elektronenstrahles in der Tragkonstruktion möglich.

Weiterhin ist es vorteilhaft, wenn die Strahlführung Mittel zur Ablenkung des Elektronenstrahles auf die Strahlerköpfe beinhaltet. Diese sind vorzugsweise als Kickvorrichtung der Umlenkmagnete ausgeführt. Der Aufbau der Strahlführung kann hierdurch vereinfacht werden. Es befindet sich somit an jedem Eckpunkt des Polygons ein Umlenkmagnet, welcher den Strahl in Richtung des nächsten Umlenkmagneten umlenkt bzw. mittels seiner Kickvorrichtung den Elektronenstrahl in Richtung auf das Isozentrum der Vorrichtung ablenkt.

Die abgelenkten Elektronenstrahlen können hierbei direkt auf das Zielvolumen gerichtet werden oder aber nach einer bevorzugten Ausführung der Erfindung auf ein Bremstarget gerichtet werden, so dass hochenergetische Röntgenstrahlung erzeugt wird. Den Strahlerköpfen ist hierzu jeweils ein Bremstarget zugeordnet.

Daneben ist es vorteilhaft, wenn den Strahlerköpfen jeweils ein Primärkollimator zugeordnet ist, mit welchem der Querschnitt des Strahles begrenzt werden kann und der Strahl hierdurch geformt werden kann.

Weiterhin ist es vorteilhaft, wenn den Strahlerköpfen jeweils ein vorzugsweise ansteuerbarer Multilamellenkollimator zugeordnet ist. Hierdurch ist es in besonders einfacher Weise möglich, die Strahlungsmenge zu regulieren. Dies ermöglicht auch eine intensitätsmodulierte Strahlenbehandlung.

Zur Messung der Photonenfluenz an den Strahlerköpfen ist es vorteilhaft, wenn den Strahlerköpfen jeweils eine Durchstrahlungskammer zugeordnet ist. Hierdurch ist eine messtechnische Erfassung der Strahlungsmenge möglich, so dass die Intensitätsmodulation in bekannter Weise geregelt durchgeführt werden kann. Vorzugsweise sind die Durchstrahlungskammern jeweils an dem Austrittsfenster der Primärkollimatoren angeordnet und direkt mit diesen verbunden.

Weiterhin ist es vorteilhaft, wenn die Durchstrahlungskammern in Sektoren unterteilt sind. Hierdurch ist eine genaue Analyse des erzeugten Strahlungsfeldes in Bezug auf die Homogenität und Strahlungsverteilung möglich.

Nach einer bevorzugten Weiterbildung der Erfindung wird der Elektronenstrahl durch die Elektronenquelle tangential in die polygonale Strahlführung eingeleitet. Durch die tangentiale Einspeisung des Elektronenstrahles in die Strahlführung kann die Ausdehnung der Tragkonstruktion bzw. der Vorrichtung in axialer Richtung der Ringstruktur weiter geringgehalten werden. Neben der ringförmigen Strahlführung trägt somit auch die tangentiale Einleitung zu einer kompakten Bauweise der Vorrichtung bei.

Weiterhin ist es vorteilhaft, wenn der Elektronenquelle wenigstens eine Umlenkvorrichtung für den Elektronenstrahl zugeordnet ist. Vorzugsweise lenkt die Umlenkvorrichtung oder die Umlenkvorrichtungen den Elektronenstrahl vor seiner Einleitung in die Strahlführung um 270° um. Bevorzugt ist die Umlenkvorrichtung derart ausgebildet, dass sie eine doppelte Fokussierung sowohl in der räumlichen Richtung als auch in der Energieverteilung des Elektronenstrahls bewirkt.

Vorteilhaft ist es weiterhin, wenn die Elektronenquelle ein Linearbeschleuniger ist. Ist eine Beschleunigungsstrecke des Linearbeschleunigers in der Ebene des Polygons angeordnet, kann der Aufbau der Vorrichtung besonders kompakt gehalten werden.

Vorteilhaft ist es weiterhin, wenn die Tragkonstruktion im wesentlichen ringförmig ausgebildet ist. Bevorzugt ist die Tragkonstruktion zweiteilig mit einem inneren und einem äußeren Tragrahmen ausgebildet. Die Strahlerköpfe sind hierbei an dem inneren Tragrahmen fixiert.

Nach einer Weiterbildung der Erfindung ist es vorteilhaft, wenn der innere Tragrahmen mit den darin fixierten Strahlerköpfen rotierbar in dem äußeren Tragrahmen gelagert ist. Hierdurch können weitere Strahlrichtungen ermöglicht werden. Vorteilhafterweise ist der innere Tragrahmen auf Rollen in dem äußeren Tragrahmen gelagert ist, um eine einfache Rotation zu ermöglichen.

Ist der innere Tragrahmen zweiteilig mit einem inneren und einem äußeren Ring ausgebildet, ist die Anordnung der strahlungsführenden und -erzeugenden Teile in besonders einfacher Weise möglich. Hierzu können beispielsweise entsprechende Ausnehmungen in den Ringen für die Strahlerköpfe oder Kollimatoren ausgeführt sein.

Sind fünf Strahlerköpfe vorgesehen und dementsprechend die Strahlführung als Fünfeck ausgebildet, ist der innere Tragrahmen vorzugsweise um einen Winkel von +/- 36° rotierbar. Hierdurch ist jede beliebige Strahlrichtung möglich, ohne die Justage der Strahlerköpfe zu gefährden. Durch die Abdeckung jeder Strahlrichtung im Vollkreis ist eine Optimierung der Dosisverteilung im Patienten möglich, wodurch gesundes Gewebe weiter geschont werden kann. Daneben ist es möglich, ohne wesentlichen Aufwand unterschiedliche Strahlrichtungen zu realisieren, wobei keine erneute Justage der Strahlerköpfe nötig ist.

Durch die erfindungsgemäße Anordnung der Strahlführung in einem Polygon, insbesondere in einem Fünfeck, lassen sich Strahlerköpfe und Kollimatoren auf vergleichsweise geringem Raum anordnen, so dass der Durchmesser der Patientenöffnung größer als in Vorrichtungen des Standes der Technik ausgeführt werden kann. Vorzugsweise beträgt der Durchmesser der Patientenöffnung zwischen 80 cm und 120 cm. Hierdurch wird für den Patiententisch ein sehr großer Freiraum gewonnen. Außerdem ist eine Positionierung des Patienten in der Weise möglich, dass das Zentrum des Behandlungsvolumens mit dem Isozentrum der Vorrichtung zur Deckung kommt.

Durch die erfindungsgemäße Anordnung der Strahlführung und der Strahlerköpfe mit Kollimatoren ist es weiterhin möglich, die axiale Ausdehnung der Vorrichtung gering zu halten. Bevorzugt weist die gesamte Tragkonstruktion mit innerem und äußerem Tragrahmen in axialer Richtung eine Ausdehnung von etwa 80 cm auf.

Die geringe Tiefe der ringförmigen Tragkonstruktion in Verbindung mit dem großen Durchmesser der Patientenöffnung ermöglicht eine vergleichsweise große Bewegbarkeit des Patiententisches. Vorzugsweise ist ein Patiententisch der Vorrichtung in horizontaler und/oder vertikaler Richtung gegenüber der Tragkonstruktion verstellbar.

Daneben ist es vorteilhaft, wenn der Patiententisch schwenkbar in der Vorrichtung angeordnet ist. Bevorzugt ist der Patiententisch um eine Horizontalachse, die das Isozentrum schneidet, schwenkbar. Insbesondere ist es vorteilhaft, wenn der Patiententisch in einer horizontalen Ebene um bis zu +/-54° schwenkbar ist. Dies erlaubt eine nichtkoplanare Strahlenbehandlung, die insbesondere bei der intrakraniellen stereotaktischen Strahlführung bis hin zur Radiochirurgie genutzt werden kann. Hierdurch lassen sich gegenüber der koplanaren Technik überlegene Dosisverteilungen realisieren.

Vorzugsweise ist der Patiententisch als Sechs-Achsentisch ausgebildet. Er ermöglicht somit beliebige Positionierungen des Zielvolumens. Durch die große Patientenöffnung sind verschiedene Verstellungen möglich, ohne dass der Patient mit der Verkleidung der Tragkonstruktion in Berührung kommt.

Ist der Patiententisch als Sechs-Achsentisch ausgebildet, ist zugleich eine vollautomatische Korrektur der Patientenposition in Bezug auf das Isozentrum der Vorrichtung möglich.

Insbesondere ist es vorteilhaft, wenn die Vorrichtung zur vollautomatischen Positionskorrektur über ein optisches Abtastungssystem verfügt. Durch einen Vergleich ermittelten Oberflächenkoordinaten mit den Koordinaten aus einem Planungs-CT können die nötigen Verschiebungsvektoren zur Positionskorrektur automatisch ermittelt und durch den Tisch ausgeführt werden.

Um eine bildunterstützte Strahlentherapie zu ermöglichen, ist es gemäß einer vorteilhaften Weiterbildung der Erfindung möglich, die Vorrichtung mit einem weiteren Gerät, beispielsweise einem Computertomographen, vorzugsweise in einer Baueinheit, zu verbinden. Ebenso kann jedoch auch ein separates Gerät auf Rollen als "Sliding Gantry" an die Vorrichtung zur Bestrahlung von Gewebe angedockt werden.

Weiterhin ist es vorteilhaft, wenn in der Tragkonstruktion jeweils gegenüber der Strahlerköpfe strahlenabsorbierende Massen angeordnet sind. Hierdurch ist es möglich, einen inneren Strahlenschutz zu realisieren, was dünnere Bunkerbauteile ermöglicht. Ebenso ist es vorteilhaft, wenn der innere Tragrahmen und/ oder der äußere Tragrahmen zumindest teilweise aus einem strahlenabsorbierenden Material bestehen.

Nach einer weiteren Ausführungsform der Erfindung ist die kinetische Energie der Elektronen des Elektronenstrahles einstellbar. Ebenso kann die Vorrichtung auch zwei Elektronenquellen umfassen, wobei die kinetische Energie der erzeugten Elektronen unterschiedlich ist.

Weitere Vorteile der Erfindung werden anhand der nachfolgend dargestellten Ausführungsbeispiele beschrieben. Es zeigen:
- **Figur 1**: eine schematische Schnittdarstellung durch eine Vorrichtung zur Bestrahlung von Gewebe mit Tragkonstruktion, Strahlführung und Strahlerköpfen,
- **Figur 2**: eine schematische Darstellung eines Querschnittes durch die Tragkonstruktion der Vorrichtung,
- **Figur 3**: eine perspektivische Darstellung des inneren Tragrahmens der Tragkonstruktion
- **Figur 4**: eine perspektivische Darstellung des äußeren Rings eines zweiteiligen inneren Tragrahmens.

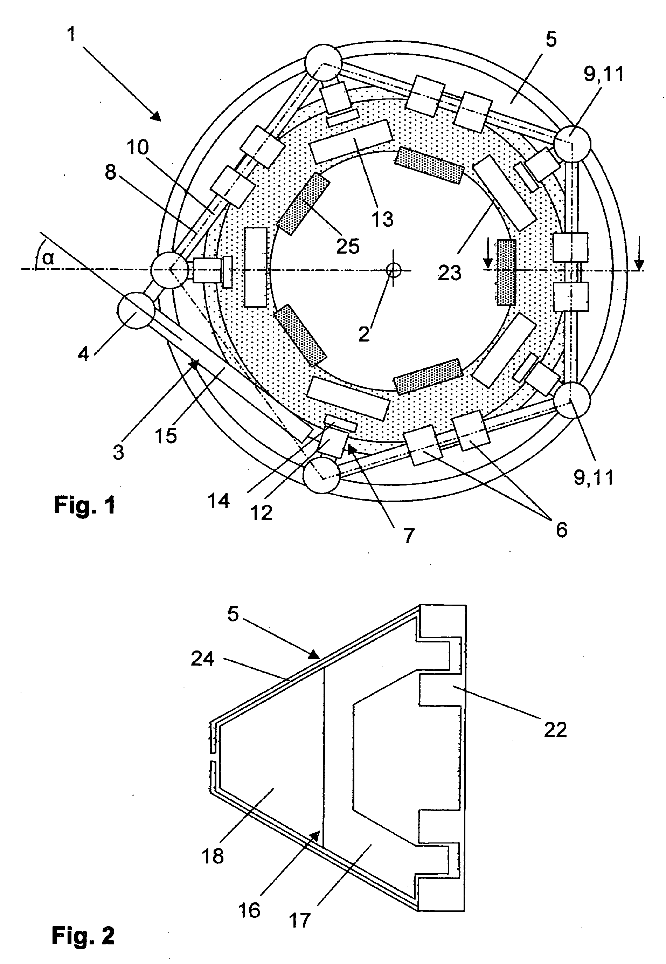

Figur 1 zeigt eine Vorrichtung 1 zur Bestrahlung von Gewebe, welche insbesondere zur Tumorbestrahlung einsetzbar ist. Hierzu ist im Inneren der weitgehend ringförmig ausgeführten Vorrichtung 1 ein Patient auf einem hier nicht dargestellten Patientenlagerungstisch derart platzierbar, dass sich das zu bestrahlende Zielvolumen in der Mitte der Vorrichtung 1, dem Isozentrum 2, befindet. Die Vorrichtung 1 weist eine Elektronenquelle 3 auf, welche vorliegend als Linearbeschleuniger ausgeführt ist. Der in der Elektronenquelle 3 erzeugte Elektronenstrahl wird in einer Umlenkvorrichtung 4 gemäß der vorliegenden Darstellung umgelenkt und schließlich tangential in eine weitgehend ringförmig ausgeführte Tragkonstruktion 5 der Vorrichtung 1 eingespeist. Vorteilhaft ist die Umlenkung um insgesamt 270°, wobei der Umlenkmagnet bei entsprechender Ausführung eine sogenannte doppelte Fokussierung sowohl in der räumlichen Richtung als auch in der Energieverteilung des Elektronenstrahls bewirkt.

Weiterhin ist in der Tragkonstruktion 5 eine Mehrzahl von Strahlerköpfen 7 feststehend und weitgehend ringförmig um das Isozentrum 2 der Vorrichtung 1 angeordnet. Die Strahlerköpfe 7 umfassen jeweils zumindest die Primärkollimatoren 12 und ggf. ein Bremstarget (hier nicht dargestellt) und geben Strahlung in Richtung auf das Isozentrum 2 der Vorrichtung 1 ab. Erfindungsgemäß ist vorgesehen, einen in die Tragkonstruktion 5 eingespeisten Elektronenstrahl 8 durch Mittel zur Umlenkung des Elektronenstrahles 8 derart in der Tragkonstruktion 5 zu führen und umzulenken, dass sich eine polygonale Strahlführung, vorliegend in Form eines Fünfeckes, ergibt. Hierzu sind an jedem Eckpunkt des Polygons 10 Mittel zur Umlenkung des Elektronenstrahles angeordnet, welche vorliegend als Umlenkmagnete 9 ausgebildet sind. Vorzugsweise ist die Strahlführung als symmetrisches Polygon 10 ausgebildet. Eine asymmetrische Ausbildung ist jedoch ebenfalls denkbar. Weiterhin sind zur Strahlführung strahlenoptische Elemente 6 jeweils zwischen den Umlenkvorrichtungen angeordnet.

Um den Elektronenstrahl 8 auf die einzelnen Strahlerköpfe 7 zu leiten, sind weiterhin Mittel zur Ablenkung des Elektronenstrahles auf die Strahlerköpfe 7 vorgesehen. Im vorliegenden Beispiel sind die Mittel zur Umlenkung des Elektronenstrahles als Umlenkmagnete 9 ausgebildet, welche zugleich über eine Kickvorrichtung 11 verfügen, um den Elektronenstrahl 8 auf die Strahlerköpfe 7 abzulenken.

Durch die erfindungsgemäße Strahlführung in Form eines Polygons 10, insbesondere eines Fünfeckes, ist es möglich, die Tragkonstruktion 5 der Vorrichtung 1 mit einer vergleichsweise geringen axialen Ausdehnung bereitzustellen, so dass ein einfacher und kompakter Aufbau der Vorrichtung 1 ermöglicht ist. Zur Realisierung mehrerer Bestrahlungsrichtungen ist keine Rotation der Strahlerköpfe 7 um das Isozentrum 2 erforderlich. Zugleich ist es durch die erfindungsgemäße ringförmige Strahlführung in Form eines Polygons 10 möglich, mehrere Strahlerköpfe 7 mit nur einer einzigen Elektronenquelle 3 zu versorgen. Hierdurch kann der Aufbau der Vorrichtung 1 weiterhin sehr kompakt gehalten werden.

In der gezeigten Darstellung sind die Umlenkmagnete 9 nacheinander ansteuerbar, so dass die Strahlerköpfe 7 nacheinander separat angesteuert werden. Ebenso ist es jedoch auch möglich, an den Eckpunkten des Polygons 10 eine Strahlteilung vorzusehen, wobei ein Teil des Strahles in Richtung auf einen Strahlerkopf 7 abgelenkt wird, während der weitere Strahl durch den Umlenkmagneten 9 in Richtung auf den nächsten Eckpunkt des Polygons 10 abgelenkt wird.

Der auf die Strahlerköpfe 7 abgelenkte Elektronenstrahl 8 kann nach entsprechender Strahlformung direkt als Nutzstrahlung auf das Zielvolumen im Isozentrum 2 der Vorrichtung 1 gerichtet werden; ebenso ist es jedoch auch möglich, den Strahlerköpfen 7 jeweils ein Bremstarget (hier nicht dargestellt) zur Erzeugung von Röntgenstrahlung zuzuordnen.

Den Strahlerköpfen 7 ist jeweils ein Primärkollimator 12 zugeordnet, um den Strahl zu formen und auf das Zielvolumen einzugrenzen. Um einen intensitätsmodulierte Bestrahlung durchführen zu können (IMRT), sind nach dem gezeigten Beispiel den Strahlerköpfen 7 weiterhin jeweils ansteuerbare Multilamellenkollimatoren 13 zugeordnet. Mittels dieser lassen sich unregelmäßige Feldformen erzeugen, so dass die Dosisverteilung in optimaler Weise an das Zielvolumen angepasst werden kann.

Um die Strahlungsmenge zu regeln, sind weiterhin an den Austrittsfenstern der Primärkollimatoren 12 Durchstrahlungskammern 14 angeordnet. Diese sind vorzugsweise direkt mit den Primärkollimatoren 12 verbunden. Die Durchstrahlungskammern 14 sind in mehrere Sektoren unterteilt, so dass die bestrahlte Dosis und auch die Homogenität und Gleichverteilung des Strahlungsfeldes ermittelt werden kann. Abweichungen von den zuvor eingestellten Sollwerten können somit erfasst und korrigiert werden.

Durch die tangentiale Einspeisung des Elektronenstrahles 8 in die Tragkonstruktion 5 kann die Vorrichtung 1 weitgehend kompakt gehalten werden. Die kompakte Bauweise der Vorrichtung 1 kann weiterhin unterstützt werden, indem die Beschleunigungsstrecke 15 des Linearbeschleunigers 3 derart in der Ebene des Polygons angeordnet ist, dass eine direkte tangentiale Einspeisung möglich ist. Im gezeigten Beispiel ist der erste Strahlerkopf so angeordnet, dass seine Strahlrichtung exakt in der Horizontalen ausgerichtet ist. Die Beschleunigungsstrecke 15 ist daher in einem Winkel α von 36° (bei der vorliegenden fünfeckigen Strahlführung) gegenüber der Horizontalen angeordnet. An dem ersten Umlenkmagneten 9 findet somit nur die Ablenkung des Elektronenstrahles 8 auf den Strahlerkopf 7 hin statt.

Die Tragkonstruktion 5, in welcher die Strahlerköpfe 7 feststehend angeordnet sind, ist im wesentlichen als ringförmige Struktur ausgebildet. Die Tragkonstruktion 5 ist vorzugsweise zweiteilig mit einem inneren Tragrahmen 16 und einem äußeren Tragrahmen 22 ausgebildet (Fig. 2). Vorzugsweise besteht der innere Tragrahmen 16 aus einem äußeren Ring 17 und einem inneren Ring 18.

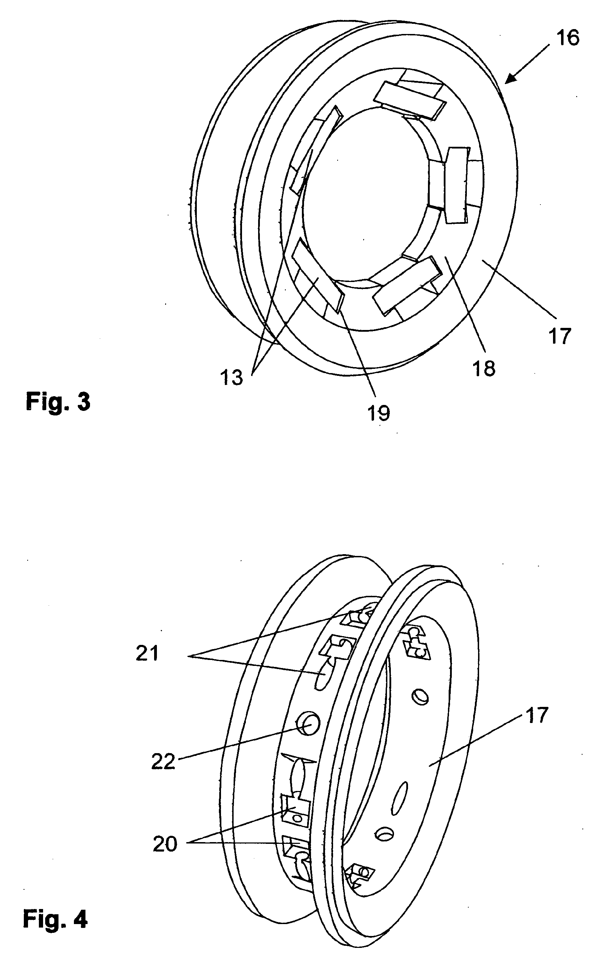

Figur 3 zeigt eine perspektivische Darstellung des inneren Tragrahmens 16 der Tragkonstruktion. In dem äußeren und inneren Ring 17, 18 sind jeweils verschiedene Aussparungen 19 angeordnet, in welche die Bauteile für Strahlführung und die Strahlerköpfe einbringbar sind. Wie aus der Darstellung der Figur 4 ersichtlich, sind in dem äußeren Ring 17 Aussparungen 20 für die strahlenoptischen Elemente 6, Aussparungen die Strahlführung in Form eines Fünfeckes 21 sowie Aussparungen 22 für die Primärkollimatoren 12 eingebracht.

In dem inneren Ring 18 sind hingegen vorwiegend Aussparungen 19 für die Multilamellenkollimatoren 13 vorgesehen.

Um bzgl. der Strahlrichtung, welche mittels der Strahlerköpfe 7 realisierbar ist, einen Vollkreis abzudecken, kann der innere Tragrahmen 16 bestehend aus dem inneren und äußeren Ring 17 und 18 drehbar in einem im wesentlichen ringförmigen äußeren Tragrahmen 22 angeordnet werden, wie in Figur 2 ersichtlich. Vorzugsweise ist der innere Tragrahmen 16 auf hier nicht dargestellten Rollen in dem äußeren Tragrahmen 22 gelagert und im Fall der vorliegenden fünfeckigen Strahlführung um einen Winkel von +/- 36° rotierbar.

Durch die erfindungsgemäße tangentiale Einspeisung des Elektronenstrahles 8 und ringförmige Strahlführung in Form eines Polygons 10 kann die Ringstruktur der Tragkonstruktion 5 sehr kompakt ausgeführt werden, so dass ein erheblicher Freiraum für den Patiententisch gewonnen wird. So kann der Durchmesser der Patientenöffnung 23 bis zu 120 cm betragen und übertrifft somit alle vergleichbaren Geräte des Standes der Technik, bei welchen der Durchmesser für die Patientenöffnung lediglich bei etwa 80 cm liegt. In axialer Richtung weist dieTragkonstruktion 5, wie in Figur 2 ersichtlich, ebenfalls nur eine geringe Ausdehnung von etwa 80 cm auf. Zusätzlich ist es durch die erfindungsgemäße Anordnung der Strahlführung und Strahlerköpfe 7 möglich, die Tragkonstruktion 5 zur Patientenöffnung 23 hin zu verjüngen, so dass sich große Bewegungsmöglichkeiten für den Patiententisch ergeben. Der Patiententisch kann somit in horizontaler und vertikaler Richtung bewegt werden, ohne dass der Patient mit der Verkleidung 24 der Tragkonstruktion 5 in Berührung kommt. Durch die verjüngte Verkleidung 24 ist es möglich, den Patiententisch in einer horizontalen Ebene um bis zu +/-54° zu verschwenken, so dass auch eine nichtkoplanare Strahlenbehandlung ermöglicht wird. Die Abmessungen der erfindungsgemäßen Vorrichtung 1 zur Bestrahlung von Gewebe ermöglichen auch die Ausbildung des Patiententisches als Sechs-Achsentisch, so dass zusätzlich ein Rollwinkel und Kippwinkel für den Tisch realisiert werden können.

Die Ausführung des Patiententisches als Sechs-Achsentisch ermöglicht auch eine vollautomatische Korrektur in der Position des Patienten. Hierzu ist die Vorrichtung 1 beispielsweise mit einem optischen Abtastungssystem nach DE 198 05 917 ausgestattet. Hierbei werden Oberflächenkoordinaten des Patienten in der aktuellen Position mit den Ausgangswerten des Planungs-CTs verglichen, woraus schließlich die sechs Verschiebungsvektoren für den Patiententisch gewonnen werden. Die Korrektur der Patientenposition ist mittels dieses Systems in kurzer Zeit durchführbar und für den Patienten absolut unschädlich. Die automatische Positionskorrektur ermöglicht zumindest näherungsweise eine bildgestützte Bestrahlung. Aufwendungen für weitere CT-Messungen können hierdurch reduziert werden.

Da die Tragkonstruktion 5 der erfindungsgemäßen Vorrichtung 1 zur Bestrahlung des Gewebes in der axialen Ausdehnung sehr kompakt ist, kann die Vorrichtung 1 problemlos mit einem weiteren Gerät, beispielsweise einem Computertomographen, verbunden werden. Hierdurch ist eine bildgestützte Bestrahlung (Image Guided Radiation Therapy) uneingeschränkt realisierbar. Der Computertomograph kann hierbei als ,,Sliding Gantry" auf Rollen an die Vorrichtung 1 angedockt werden oder aber auch mit dieser in einer Baueinheit verbunden sein.

Um die aus der Vorrichtung 1 austretende Strahlung zu reduzieren, ist es zweckmäßig, die Vorrichtung 1 mit Strahlstoppern für inneren Strahlenschutz auszustatten. Der innere Tragrahmen 16 bzw. der innere Ring 18 und äußere Ring 17 können hierzu aus einem strahlenabsorbierenden Material gefertigt sein. Für das Material kommen Stahl, Woodmetall, Blei oder ähnlich schwere Stoffe in Frage. So kann der Tragrahmen 16 beispielsweise aus duktilem Kugelgraphitguss hergestellt werden, da dieser mechanisch belastbar, schweißbar und gut mechanisch bearbeitbar ist. Ebenso ist es möglich, in der Tragkonstruktion 5 jeweils gegenüber der Strahlerköpfe 7 strahlenabsorbierende Massen 25 anzuordnen (s. Fig. 1). Weiterhin ist es möglich, zumindest Teile des äußeren Tragrahmens 22 aus einem strahlenabsorbierenden Material herzustellen.

Nach einer weiteren, hier nicht dargestellten Variante, können als strahlenabsorbierende Massen 25 beispielsweise Stahlklötze an dem inneren Ring 17 aufgesattelt werden, welche gleichzeitig einen seitlichen Einschub für die Multilamellenkollimatoren 13 vorsehen.

Die Erfindung ist nicht auf die dargestellten Ausführungsbeispiele beschränkt. Abwandlungen und Kombinationen im Rahmen der Patentansprüche fallen ebenfalls unter die Erfindung.

## Patentansprüche

1. Vorrichtung (1) zur Bestrahlung von Gewebe, mit mindestens einer Elektronenquelle (3) zur Erzeugung eines Elektronenstrahls (8), mit einer Mehrzahl von Strahlerköpfen (7), welche in einer Tragkonstruktion (5) feststehend und ringförmig um ein Isozentrum (2) der Vorrichtung (1) angeordnet sind und Strahlung in Richtung auf das Isoz-entrum (2) abgeben, und mit einer Strahlführung, mittels welcher der Elektronenstrahl (8) in der Tragkonstruktion (5) geführt und auf die Strahlerköpfe (7) geleitet wird, **dadurch gekennzeichnet, dass** die Strahlführung als Polygon (10), insbesondere als Fünfeck, ringförmig um das Isozentrum (2) ausgebildet ist, wobei an jedem Eckpunkt des Polygons (10) Mittel zur Umlenkung des Elektronenstrahls (8) angeordnet sind.

2. Vorrichtung zur Bestrahlung von Gewebe nach dem vorherigen Anspruch, **dadurch gekennzeichnet, dass** für alle Strahlerköpfe (7) eine gemeinsame Elektronenquelle (3) vorgesehen ist.

3. Vorrichtung zur Bestrahlung von Gewebe nach einem der vorherigen Ansprüche, **dadurch gekennzeichnet, dass** die Strahlführung als symmetrisches Polygon (10) ausgebildet ist.

4. Vorrichtung zur Bestrahlung von Gewebe nach einem der vorherigen Ansprüche, **dadurch gekennzeichnet, dass** die Mittel zur Umlenkung des Elektronenstrahls (8) als Umlenkmagnete (9) ausgebildet sind.

5. Vorrichtung zur Bestrahlung von Gewebe nach einem der vorherigen Ansprüche, **dadurch gekennzeichnet, dass** die Strahlführung Mittel zur Ablenkung des Elektronenstrahls (8) auf die Strahlerköpfe (7) beinhaltet.

6. Vorrichtung zur Bestrahlung von Gewebe nach einem der vorherigen Ansprüche, **dadurch gekennzeichnet, dass** die Umlenkmagnete (9) eine Kickvorrichtung (11) zur Ablenkung des Elektronenstrahls (8) auf die Strahlerköpfe (7) aufweisen.

7. Vorrichtung zur Bestrahlung von Gewebe nach einem der vorherigen Ansprüche, **dadurch gekennzeichnet, dass** den Strahlerköpfen (7) jeweils ein Bremstarget zur Erzeugung von Röntgenstrahlung zugeordnet ist.

8. Vorrichtung zur Bestrahlung von Gewebe nach einem der vorherigen Ansprüche, **dadurch gekennzeichnet, dass** den Strahlerköpfen (7) jeweils ein Primärkollimator (12)zugeordnet ist.

9. Vorrichtung zur Bestrahlung von Gewebe nach einem der vorherigen Ansprüche, **dadurch gekennzeichnet, dass** den Strahlerköpfen (7) jeweils ein vorzugsweise ansteuerbarer Multilamellenkollimator (13) zugeordnet ist.

10. Vorrichtung zur Bestrahlung von Gewebe nach einem der vorherigen Ansprüche, **dadurch gekennzeichnet, dass** den Strahlerköpfen (7) jeweils eine Durchstrahlungskamrner (14) zugeordnet ist.

11. Vorrichtung zur Bestrahlung von Gewebe nach einem der vorherigen Ansprüche, **dadurch gekennzeichnet, dass** die Durchstrahlungskammern (14) direkt mit den Primärkollimatoren (12) verbunden sind.

12. Vorrichtung zur Bestrahlung von Gewebe nach einem der vorherigen Ansprüche, **dadurch gekennzeichnet, dass** die Durchstrahlungskammern (14) in Sektoren unterteilt sind.

13. Vorrichtung zur Bestrahlung von Gewebe nach einem der vorherigen Ansprüche, **dadurch gekennzeichnet, dass** der Elektronenstrahl (8) durch die Elektronenquelle (3) tangential in das Polygon (10) der Strahlführung einleitbar ist.

14. Vorrichtung zur Bestrahlung von Gewebe nach einem der vorherigen Ansprüche, **dadurch gekennzeichnet, dass** der Elektronenquelle (3) wenigstens eine Umlenkvorrichtung (4) für den Elektronenstrahl (8) zugeordnet ist.

15. Vorrichtung zur Bestrahlung von Gewebe nach einem der vorherigen Ansprüche, **dadurch gekennzeichnet, dass** die Umlenkvorrichtung(en) (4) den Elektronenstrahl (8) vor seiner Einleitung in das Polygon (10) um 270° umlenken.

16. Vorrichtung zur Bestrahlung von Gewebe nach einem der vorherigen Ansprüche, **dadurch gekennzeichnet, dass** die Umlenkvorrichtung(en) (4) als doppelt fokussierende Umlenkvorrichtung(en) (4) ausgebildet ist/sind.

17. Vorrichtung zur Bestrahlung von Gewebe nach einem der vorherigen Ansprüche, **dadurch gekennzeichnet, dass** die Elektronenquelle (3) ein Linearbeschleuniger ist.

18. Vorrichtung zur Bestrahlung von Gewebe nach einem der vorherigen Ansprüche, **dadurch gekennzeichnet, dass** eine Beschleunigungsstrecke (15) des Linearbeschleunigers in der Ebene des Polygons (10) angeordnet ist.

19. Vorrichtung zur Bestrahlung von Gewebe nach einem der vorherigen Ansprüche, **dadurch gekennzeichnet, dass** die Tragkonstruktion (5) im wesentlichen ringförmig ausgebildet ist.

20. Vorrichtung zur Bestrahlung von Gewebe nach einem der vorherigen Ansprüche, **dadurch gekennzeichnet, dass** die Tragkonstruktion (5) zweiteilig mit einem inneren (16) und einem äußeren Tragrahmen (22) ausgebildet ist.

21. Vorrichtung zur Bestrahlung von Gewebe nach einem der vorherigen Ansprüche, **dadurch gekennzeichnet, dass** der innere Tragrahmen (16) mit den darin fixierten Strahlerköpfen (7) rotierbar in dem äußeren Tragrahmen (22) angeordnet ist.

22. Vorrichtung zur Bestrahlung von Gewebe nach einem der vorherigen Ansprüche, **dadurch gekennzeichnet, dass** der innere Tragrahmen (16) auf Rollen in dem äußeren Tragrahmen (22) gelagert ist.

23. Vorrichtung zur Bestrahlung von Gewebe nach einem der vorherigen Ansprüche, **dadurch gekennzeichnet, dass** der innere Tragrahmen (16) zweiteilig mit einem inneren (18) und einem äußeren (17) Ring ausgebildet ist.

24. Vorrichtung zur Bestrahlung von Gewebe nach einem der vorherigen Ansprüche, **dadurch gekennzeichnet, dass** der innere Tragrahmen (16) um einen Winkel von +/- 36° rotierbar ist.

25. Vorrichtung zur Bestrahlung von Gewebe nach einem der vorherigen Ansprüche, **dadurch gekennzeichnet, dass** der Durchmesser der Patientenöffnung (23) der Vorrichtung zwischen 80 cm und 120 cm beträgt.

26. Vorrichtung zur Bestrahlung von Gewebe nach einem der vorherigen Ansprüche, **dadurch gekennzeichnet, dass**die gesamte Tragkonstruktion (5) mit innerem (16) und äußerem Tragrahmen (22) in axialer Richtung eine Ausdehnung von etwa 80 cm aufweist.

27. Vorrichtung zur Bestrahlung von Gewebe nach einem der vorherigen Ansprüche, **dadurch gekennzeichnet, dass** ein Patiententisch der Vorrichtung (1) in horizontaler und/oder vertikaler Richtung gegenüber der Tragkonstruktion (5) verstellbar ist.

28. Vorrichtung zur Bestrahlung von Gewebe nach einem der vorherigen Ansprüche, **dadurch gekennzeichnet, dass** der Patiententisch schwenkbar in der Vorrichtung (1) angeordnet ist.

29. Vorrichtung zur Bestrahlung von Gewebe nach einem der vorherigen Ansprüche, **dadurch gekennzeichnet, dass** der Patiententisch in einer horizontalen Ebene um bis zu +/- 54° schwenkbar ist.

30. Vorrichtung zur Bestrahlung von Gewebe nach einem der vorherigen Ansprüche, **dadurch gekennzeichnet, dass** der Patiententisch als 6-Achsentisch ausgebildet ist.

31. Vorrichtung zur Bestrahlung von Gewebe nach einem der vorherigen Ansprüche, **dadurch gekennzeichnet, dass** die Position des Patienten in Bezug zu dem Isozentrum (2) der Vorrichtung (1) vollautomatisch korrigierbar ist.

32. Vorrichtung zur Bestrahlung von Gewebe nach einem der vorherigen Ansprüche, **dadurch gekennzeichnet, dass** zur vollautomatischen Positionskorrektur die Vorrichtung (1) über ein optisches Abtastungssystem verfügt.

33. Vorrichtung zur Bestrahlung von Gewebe nach einem der vorherigen Ansprüche, **dadurch gekennzeichnet, dass** die Vorrichtung (1) mit einem weiteren Gerät, beispielsweise einem Computertomographen, vorzugsweise in einer Baueinheit verbunden ist.

34. Vorrichtung zur Bestrahlung von Gewebe nach einem der vorherigen Ansprüche, **dadurch gekennzeichnet, dass** in der Tragkonstruktion (5) jeweils gegenüber der Strahlerköpfe (7) strahlenabsorbierende Massen (25) angeordnet sind.

35. Vorrichtung zur Bestrahlung von Gewebe nach einem der vorherigen Ansprüche, **dadurch gekennzeichnet, dass** der innere Tragrahmen (16) und/ oder der äußere Tragrahmen (22) zumindest teilweise aus einem strahlenabsorbierenden Material bestehen.

36. Vorrichtung zur Bestrahlung von Gewebe nach einem der vorherigen Ansprüche, **dadurch gekennzeichnet, dass** die kinetische Energie der Elektronen des Elektronenstrahls (8) einstellbar ist.

37. Vorrichtung zur Bestrahlung von Gewebe nach einem der vorherigen Ansprüche, **dadurch gekennzeichnet, dass** die Vorrichtung (1) zwei Elektronenquellen (3) umfasst, wobei die kinetische Energie der erzeugten Elektronen unterschiedlich ist.

## Claims

1. Device (1) for irradiating tissue with at least one electron source (3) for generating an electron beam (8), with numerous radiation heads (7) that are firmly and annularly arranged around an isocenter (2) of the device (1) in a supporting structure (5) and emit radiation towards the isocenter (2), and one beam guidance system to guide the electron beam (8) in the supporting structure (5) towards the radiation heads (7) **characterized in that** the beam guidance system has been built as a ring-shaped polygon (10), especially as a pentagon, around the isocenter (2), in which case media for deflecting the electron beam (8) have been placed at every corner point of the polygon (10).

2. Device for irradiating tissue according to the preceding claim, **characterized in that** one common electron source (3) is provided for all radiation heads (7).

3. Device for irradiating tissue according to one of the preceding claims, **characterized in that** the beam guidance system has been designed as a symmetrical polygon (10).

4. Device for irradiating tissue according to one of the preceding claims, **characterized in that** the media for deflecting the electron beam (8) have been designed as deflecting magnets (9).

5. Device for irradiating tissue according to one of the preceding claims, **characterized in that** the beam guidance system includes media for deflecting the electron beam (8) towards the radiation heads (7).

6. Device for irradiating tissue according to one of the preceding claims, **characterized in that** the deflecting magnets (9) have a kicker device (11) for deflecting the electron beam (8) to the radiation heads (7).

7. Device for irradiating tissue according to one of the preceding claims, **characterized in that** in each case, a braking target for generating X-rays has been assigned to the radiation heads (7).

8. Device for irradiating tissue according to one of the preceding claims, **characterized in that** in each case, a primary collimator (12) has been assigned to the radiation heads (7).

9. Device for irradiating tissue according to one of the preceding claims, **characterized in that** in each case, a preferably controllable multilamellar collimator (13) has been assigned to the radiation heads (7).

10. Device for irradiating tissue according to one of the preceding claims, **characterized in that** in each case, an irradiation chamber (14) has been assigned to the radiation heads (7).

11. Device for irradiating tissue according to one of the preceding claims, **characterized in that** the irradiation chambers (14) are directly connected to the primary collimators (12).

12. Device for irradiating tissue according to one of the preceding claims, **characterized in that** the irradiation chambers (14) are subdivided into sectors.

13. Device for irradiating tissue according to one of the preceding claims, **characterized in that** the electron beam (8) can be tangentially fed through the electron source (3) into the polygon (10) of the beam guidance system.

14. Device for irradiating tissue according to one of the preceding claims, **characterized in that** at least one deflection device (4) for the electron beam (8) has been assigned to the electron source (3).

15. Device for irradiating tissue according to one of the preceding claims, **characterized in that** the deflection device(s) (4) deflect the electron beam (8) by 270° before it is introduced into the polygon (10).

16. Device for irradiating tissue according to one of the preceding claims, **characterized in that** the deflection device(s) (4) has/have been designed as double-focusing deflection device(s) (4).

17. Device for irradiating tissue according to one of the preceding claims, **characterized in that** the electron source (3) is a linear accelerator.

18. Device for irradiating tissue according to one of the preceding claims, **characterized in that** an accelerating section (15) of the linear accelerator has been arranged on the plane of the polygon (10).

19. Device for irradiating tissue according to one of the preceding claims, **characterized in that** the supporting structure (5) has a largely annular shape.

20. Device for irradiating tissue according to one of the preceding claims, **characterized in that** the supporting structure (5) has been designed in two parts, an internal (16) and external supporting frame (22).

21. Device for irradiating tissue according to one of the preceding claims, **characterized in that** the internal supporting frame (16) with the radiation heads (7) fixed therein is rotatably arranged in the external supporting frame (22).

22. Device for irradiating tissue according to one of the preceding claims, **characterized in that** the internal supporting frame (16) is stored on rollers in the external supporting frame (22).

23. Device for irradiating tissue according to one of the preceding claims, **characterized in that** the internal supporting frame (16) has been designed in two parts, an internal (18) and an external (17) ring.

24. Device for irradiating tissue according to one of the preceding claims, **characterized in that** the internal supporting frame (16) can be rotated by an angle of +/- 36°.

25. Device for irradiating tissue according to one of the preceding claims, **characterized in that** the diameter for the patient's opening (23) of the device measures between 80 cm and 120 cm.

26. Device for irradiating tissue according to one of the preceding claims, **characterized in that** the entire supporting structure (5) with an internal (16) and external supporting frame (22) measures about 80 cm in axial direction.

27. Device for irradiating tissue according to one of the preceding claims, **characterized in that** the patient's table of the device (1) can be horizontally and/or vertically adjusted with respect to the supporting structure (5).

28. Device for irradiating tissue according to one of the preceding claims, **characterized in that** the patient's table can be swiveled in the device (1).

29. Device for irradiating tissue according to one of the preceding claims, **characterized in that** the patient's table can be swiveled up to +/- 54° in a horizontal plane.

30. Device for irradiating tissue according to one of the preceding claims, **characterized in that** the patient's table has been built as a 6-axis table.

31. Device for irradiating tissue according to one of the preceding claims, **characterized in that** the patient's position can be corrected fully automatically with respect to the isocenter (2) of the device (1).

32. Device for irradiating tissue according to one of the preceding claims, **characterized in that** the device (1) is equipped with an optical scanning system for fully automated positioning correction.

33. Device for irradiating tissue according to one of the preceding claims, **characterized in that** another unit, such as a computer tomograph, has been connected to the device (1), preferably in a structural unit.

34. Device for irradiating tissue according to one of the preceding claims, **characterized in that** radiation-absorbing masses (25) have been arranged in the supporting structure (5), in each case opposite the radiation heads (7).

35. Device for irradiating tissue according to one of the preceding claims, **characterized in that** the internal supporting frame (16) and/or the external supporting frame (22) are made at least partly of a radiation-absorbing material.

36. Device for irradiating tissue according to one of the preceding claims, **characterized in that** the kinetic energy of the electrons that make up the electron beam (8) can be regulated.

37. Device for irradiating tissue according to one of the preceding claims, **characterized in that** the device (1) comprises two electron sources (3), and the kinetic energy of the generated electrons differ.

## Revendications

1. Dispositif (1) pour l'irradiation d'un tissu, avec au moins un émetteur (3) d'électrons pour la génération d'un faisceau d'électrons (8), avec une multitude de têtes d'irradiation (7), lesquelles sont disposées dans une construction porteuse (5), de manière fixe et annulaire autour d'un isocentre (2) du dispositif (1), et émettent un rayonnement dans la direction de l'isocentre (2), et avec un déflecteur de faisceau d'électrons, à l'aide duquel le faisceau d'électrons (8) est guidé dans la construction porteuse (5) et dirigé vers les têtes d'irradiation (7), **caractérisé en ce que** le déflecteur de faisceau d'électrons se présente sous la forme d'un polygone (10), particulièrement d'un pentagone, disposé de manière annulaire autour de l'isocentre (2), sachant que des moyens pour la déflection du faisceau d'électrons (8) sont disposés à chaque sommet du polygone (10).

2. Dispositif pour l'irradiation d'un tissu selon la revendication précédente, **caractérisé en ce qu'**un émetteur d'électrons commun (3) est prévu pour toutes les têtes d'irradiation (7).

3. Dispositif pour l'irradiation d'un tissu selon l'une quelconque des revendications précédentes, **caractérisé en ce que** le déflecteur de faisceau se présente sous la forme d'un polygone (10) symétrique.

4. Dispositif pour l'irradiation d'un tissu selon l'une quelconque des revendications précédentes, **caractérisé en ce que** les moyens pour la déflection du faisceau d'électrons (8) se présentent sous la forme d'aimants déflecteurs (9).

5. Dispositif pour l'irradiation d'un tissu selon l'une quelconque des revendications précédentes, **caractérisé en ce que** le déflecteur de faisceau contient des moyens pour la déflection du faisceau d'électrons (8) vers les têtes d'irradiation (7).

6. Dispositif pour l'irradiation d'un tissu selon l'une quelconque des revendications précédentes, **caractérisé en ce que** les aimants déflecteurs (9) comportent un dispositif de déflection (11) pour la déflection du faisceau d'électrons (8) vers les têtes d'irradiation (7).

7. Dispositif pour l'irradiation d'un tissu selon l'une quelconque des revendications précédentes, **caractérisé en ce qu'**une cible de freinage est attribuée respectivement aux têtes d'irradiation (7) pour la génération d'un rayonnement X.

8. Dispositif pour l'irradiation d'un tissu selon l'une quelconque des revendications précédentes, **caractérisé en ce qu'**un collimateur primaire (12) est attribué respectivement aux têtes d'irradiation (7).

9. Dispositif pour l'irradiation d'un tissu selon l'une quelconque des revendications précédentes, **caractérisé en ce qu'**un collimateur à lames multiples (13), de préférence commandable, est attribué respectivement aux têtes d'irradiation (7).

10. Dispositif pour l'irradiation d'un tissu selon l'une quelconque des revendications précédentes, **caractérisé en ce qu'**une chambre d'irradiation (14) est attribuée respectivement aux têtes d'irradiation (7).

11. Dispositif pour l'irradiation d'un tissu selon l'une quelconque des revendications précédentes, **caractérisé en ce que** les chambres d'irradiation (14) sont reliées directement aux collimateurs primaires (12).

12. Dispositif pour l'irradiation d'un tissu selon l'une quelconque des revendications précédentes, **caractérisé en ce que** les chambres d'irradiation (14) sont réparties en secteurs.

13. Dispositif pour l'irradiation d'un tissu selon l'une quelconque des revendications précédentes, **caractérisé en ce que** le faisceau d'électrons (8) peut être introduit tangentiellement par l'émetteur d'électrons (3) dans le polygone (10) du déflecteur de faisceau.

14. Dispositif pour l'irradiation d'un tissu selon l'une quelconque des revendications précédentes, **caractérisé en ce qu'**un dispositif déflecteur (4) au moins est attribué à l'émetteur d'électrons (3) pour le faisceau d'électrons (8).

15. Dispositif pour l'irradiation d'un tissu selon l'une quelconque des revendications précédentes, **caractérisé en ce que** le(s) dispositif(s) déflecteur(s) (4) dévie(nt) le faisceau d'électrons (8) de 270° avant son introduction dans le polygone (10).

16. Dispositif pour l'irradiation d'un tissu selon l'une quelconque des revendications précédentes, **caractérisé en ce que** le(s) dispositif(s) déflecteur(s) (4) se présente(nt) sous la forme d'un (de) dispositif(s) déflecteur(s) (4) à double focalisation.

17. Dispositif pour l'irradiation d'un tissu selon l'une quelconque des revendications précédentes, **caractérisé en ce que** l'émetteur d'électrons (3) est un accélérateur linéaire.

18. Dispositif pour l'irradiation d'un tissu selon l'une quelconque des revendications précédentes, **caractérisé en ce qu'**un parcours d'accélération (15) de l'accélérateur linéaire est disposé dans le plan du polygone (10).

19. Dispositif pour l'irradiation d'un tissu selon l'une quelconque des revendications précédentes, **caractérisé en ce que** la construction porteuse (5) se présente sous une forme essentiellement annulaire.

20. Dispositif pour l'irradiation d'un tissu selon l'une quelconque des revendications précédentes, **caractérisé en ce que** la construction porteuse (5) se présente sous une forme en deux pièces, avec un cadre-porteur intérieur (16) et un cadre-porteur extérieur (22).

21. Dispositif pour l'irradiation d'un tissu selon l'une quelconque des revendications précédentes, **caractérisé en ce que** le cadre-porteur intérieur (16), avec les têtes d'irradiation (7) qui y sont fixées, est disposé de manière rotative dans le cadre-porteur extérieur (22).

22. Dispositif pour l'irradiation d'un tissu selon l'une quelconque des revendications précédentes, **caractérisé en ce que** le cadre-porteur intérieur (16) est logé sur des rouleaux dans le cadre-porteur extérieur (22).

23. Dispositif pour l'irradiation d'un tissu selon l'une quelconque des revendications précédentes, **caractérisé en ce que** le cadre-porteur intérieur (16) se présente sous une forme en deux pièces, se composant d'un anneau intérieur (18) et d'un anneau extérieur (17).

24. Dispositif pour l'irradiation d'un tissu selon l'une quelconque des revendications précédentes, **caractérisé en ce que** le cadre-porteur intérieur (16) est rotatif sur un angle de +/- 36°.

25. Dispositif pour l'irradiation d'un tissu selon l'une quelconque des revendications précédentes, **caractérisé en ce que** le diamètre de l'ouverture patient (23) du dispositif se situe entre 80 cm et 120 cm.

26. Dispositif pour l'irradiation d'un tissu selon l'une quelconque des revendications précédentes, **caractérisé en ce que** la construction porteuse entière (5), avec le cadre-porteur intérieur (16) et le cadre-porteur extérieur (22), présente dans le sens axial une étendue de 80 cm environ.

27. Dispositif pour l'irradiation d'un tissu selon l'une quelconque des revendications précédentes, **caractérisé en ce qu'**une table de patient du dispositif (1) est ajustable par rapport à la construction porteuse (5) dans le sens horizontal et/ou vertical.

28. Dispositif pour l'irradiation d'un tissu selon l'une quelconque des revendications précédentes, **caractérisé en ce que** la table de patient est disposée de manière pivotante dans le dispositif (1).

29. Dispositif pour l'irradiation d'un tissu selon l'une quelconque des revendications précédentes, **caractérisé en ce que** la table de patient est pivotante dans un plan horizontal dans une plage allant jusqu'à +/- 54°.

30. Dispositif pour l'irradiation d'un tissu selon l'une quelconque des revendications précédentes, **caractérisé en ce que** la table de patient se présente sous la forme d'une table à six axes.

31. Dispositif pour l'irradiation d'un tissu selon l'une quelconque des revendications précédentes, **caractérisé en ce que** la position du patient par rapport à l'isocentre (2) du dispositif (1) est corrigible de manière entièrement automatique.

32. Dispositif pour l'irradiation d'un tissu selon l'une quelconque des revendications précédentes, **caractérisé en ce que** le dispositif (1) est doté d'un système de balayage optique pour la correction de position entièrement automatique.

33. Dispositif pour l'irradiation d'un tissu selon l'une quelconque des revendications précédentes, **caractérisé en ce que** le dispositif (1) est relié à un appareil supplémentaire, par exemple un tomographe avec calculateur intégré, de préférence dans un seul ensemble de construction.

34. Dispositif pour l'irradiation d'un tissu selon l'une quelconque des revendications précédentes, **caractérisé en ce que** des masses absorbant le rayonnement (25) sont disposées dans la construction porteuse (5), respectivement en face des têtes d'irradiation (7).

35. Dispositif pour l'irradiation d'un tissu selon l'une quelconque des revendications précédentes, **caractérisé en ce que** le cadre-porteur intérieur (16) et/ou le cadre-porteur extérieur (22) se compose(nt) au moins partiellement d'un matériau absorbant le rayonnement.

36. Dispositif pour l'irradiation d'un tissu selon l'une quelconque des revendications précédentes, **caractérisé en ce que** l'énergie cinétique des électrons du faisceau d'électrons (8) est réglable.

37. Dispositif pour l'irradiation d'un tissu selon l'une quelconque des revendications précédentes, **caractérisé en ce que** le dispositif (1) comporte deux émetteurs d'électrons (3), sachant que les énergies cinétiques respectives des électrons générés sont différentes
